# EUROPEAN PATENT SPECIFICATION

(11) **EP 4 351 680 B1**
(45) Date of publication and mention of the grant of the patent: **21.10.2026**
(21) Application number: 22821105.8
(22) Date of filing: 10.06.2022
(51) Int. Cl.: A61M 5/142

(54) **USER-WEARABLE INFUSION PUMP HOLDER**
VOM BENUTZER TRAGBARER INFUSIONSPUMPENHALTER
SUPPORT DE POMPE À PERFUSION POUVANT ÊTRE PORTÉ PAR L'UTILISATEUR

(30) Priority: 10.06.2021 US 202163209210 P
(43) Date of publication of application: 17.04.2024
(73) Proprietor: Tandem Diabetes Care, Inc., San Diego, CA 92130 (US)
(72) Inventor: NADWORNY, John Charles, San Diego, CA 92121 (US); LAMB, Philip Sven, San Diego, CA 92121 (US)
(74) Representative: HGF
(86) International application number: PCT/US2022/033000
(87) International publication number: WO 2022/261431

(56) References cited:
- WO-A2-2012/108956
- US-A1- 2008 041 897
- US-A1- 2009 250 494
- US-A1- 2009 250 494
- US-A1- 2012 060 261
- US-A1- 2015 157 788
- US-A1- 2019 351 134
- US-B1- 10 279 106

## Description

### RELATED APPLICATION

This application claims the benefit of U.S. Provisional Application No. 63/209,210 filed June 10, 2021.

### TECHNICAL FIELD

The present disclosure relates to medical pumps for delivering medicament to a patient, and more specifically, to a user-wearable infusion pump that can be worn on the body of a user.

### BACKGROUND

There are a wide variety of medical treatments that include the administration of a therapeutic fluid in precise, known amounts at predetermined intervals. Devices and methods exist that are directed to the delivery of such fluids, which may be liquids or gases, are known in the art.

One category of such fluid delivery devices includes insulin injecting pumps developed for administering insulin to patients afflicted with type 1, or in some cases, type 2 diabetes. Some insulin injecting pumps are configured as portable or ambulatory infusion devices that can provide continuous subcutaneous insulin injection and/or infusion therapy as an alternative to multiple daily insulin injections via syringe or injector pen. Such ambulatory infusion pumps may be worn by the user, may use replaceable medicament cartridges, and may deliver other medicaments alone, or in combination with insulin. Such medicaments include glucagon, pramlintide, and the like. Examples of such pumps and various features associated therewith include those disclosed in U.S. Patent Publication Nos. 2013/0324928 and 2013/0053816 and U.S. Patent Nos. 8,287,495; 8,573,027; 8,986,253; and 9,381,297.

One type of pump that has been developed is a patch pump, also known as a micropump. Patch pumps are small pumps that may be carried directly on the skin under the user's clothing. In some cases, the pumps are situated directly on, or very near to, the injection site such that little or no tubing is required to deliver the insulin or other medicament to the patient. These pumps can therefore be worn directly on the body of the user or carried in close proximity to the body. Typically, patch pumps that are worn on the body are affixed to the body with an adhesive patch. Such pumps may need to be periodically removed from the body to e.g., charge a battery, refill a medicament reservoir, be replaced with a new pump, etc.

Document US 2009/250494A1 discloses a universal interface for an infusion system. Specifically it relates to a new carrier system for an infusion system with improved wearing comfort and handling.

Document US 2015/157788A1 discloses a therapeutic system configured for the delivery of fluid.

Document US 2019/351134A1 discloses a user-wearable patch pump system for delivery of insulin or other medicament which can include a pump and an attachment portion that attaches the pump to a user's body.

### SUMMARY

Embodiments of the invention are set out in the appended claims. The description and drawings may also present additional non-claimed embodiments, exemplary embodiments, examples, aspects and implementations for the better understanding of the claimed embodiments defined in the appended claims.

Embodiments of the present disclosure relate to a holder or carrier for a user-wearable infusion pump (patch pump) that can be worn on the patient's body and a corresponding system. Embodiments depicted and described herein provide a more versatile and secure way to hold and carry a user-wearable infusion pump that enables easy removal and insertion of the pump into the holder and further provides access to pump functions while the pump is in the holder.

In embodiments, a user-wearable infusion pump system includes a user-wearable infusion pump configured to contain a medicament and a retention frame including a frame base having a first side and an opposing second side and a first end and opposing second end defining a frame perimeter having a shape generally matching an outer perimeter of the user-wearable pump. An elastic sleeve can be attached to the retention frame and extend across the retention frame from the first side of the retention frame to the second side of the retention frame. The elastic sleeve can be configured to be stretched away from the retention frame to enable insertion of the user-wearable infusion pump onto the retention frame and to flex back against the user-wearable infusion pump to provide a force biasing the infusion pump on the retention frame.

In embodiments, a pump holder for selectively retaining a user-wearable infusion pump, comprising can include a retention frame including a frame base having a first side and an opposing second side and a first end and opposing second end defining a frame perimeter having a shape generally matching an outer perimeter of a user-wearable pump and an elastic sleeve attached to the retention frame and extending across the retention frame from the first side of the retention frame to the second side of the retention frame. The elastic sleeve can be configured to be stretched away from the retention frame to enable insertion of the user-wearable infusion pump onto the retention frame and to flex back against the user-wearable infusion pump to provide a force biasing the infusion pump on the retention frame.

The above summary is not intended to describe each illustrated embodiment or every implementation of the subject matter hereof. The figures and the detailed description that follow more particularly exemplify various embodiments.

### BRIEF DESCRIPTION OF THE DRAWINGS

Subject matter hereof may be more completely understood in consideration of the following detailed description of various embodiments in connection with the accompanying figures, in which:
Figs. 1A-1C depicts an embodiment of a pump system according to the disclosure.
Figs. 2A-2B depict an embodiment of a pump system according to the disclosure.
Fig. 3 depicts an embodiment of a pump system according to the disclosure.
Figs. 4A-4B depict remote control devices for a pump system according to embodiments of the disclosure.
Figs. 5A-5B depict a holder for a user-wearable infusion pump according to an embodiment.
Fig. 6 depicts a schematic representation of the holder of Figs. 5A-5B.
Fig. 7 depicts the holder of Figs. 5A-5B with a pump inserted therein.
Figs. 8A-8D depict a tray that holds a user wearable infusion pump according to an embodiment, constraining motion of the pump in the plane of the tray.
Figs. 9A-9B depict a holder for a user-wearable infusion pump according to an embodiment.
Figs. 10A-10B depict a holder for a user-wearable infusion pump according to an embodiment.
Figs. 11A-11B depict a holder for a user-wearable infusion pump according to an embodiment.
Figs. 12A-12B depict a holder for a user-wearable infusion pump according to an embodiment.
Figs. 13A-13B depict a holder for a user-wearable infusion pump according to an embodiment.
Figs. 14A-14B depict a holder for a user-wearable infusion pump according to an embodiment.
Figs. 15A-15E depict a holder for a user-wearable infusion pump according to an embodiment.
Figs. 16A-16E depict a holder for a user-wearable infusion pump according to an embodiment.
Figs. 17A-17D depict a holder for a user-wearable infusion pump according to an embodiment.
Figs. 18A-18C depict a holder for a user-wearable infusion pump according to an embodiment.
Figs. 19A-19C depict a holder for a user-wearable infusion pump according to an embodiment.
Figs. 20A-20B depict a holder for a user-wearable infusion pump according to an embodiment.
Fig. 21 depicts a tray for a holder for a user-wearable infusion pump according to an embodiment.

While various embodiments are amenable to various modifications and alternative forms, specifics thereof have been shown by way of example in the drawings and will be described in detail. It should be understood, however, that the intention is not to limit the claimed inventions to the particular embodiments described. On the contrary, the intention is to cover all modifications, equivalents, and alternatives falling within the scope of the subject matter as defined by the claims.

### DETAILED DESCRIPTION OF THE DRAWINGS

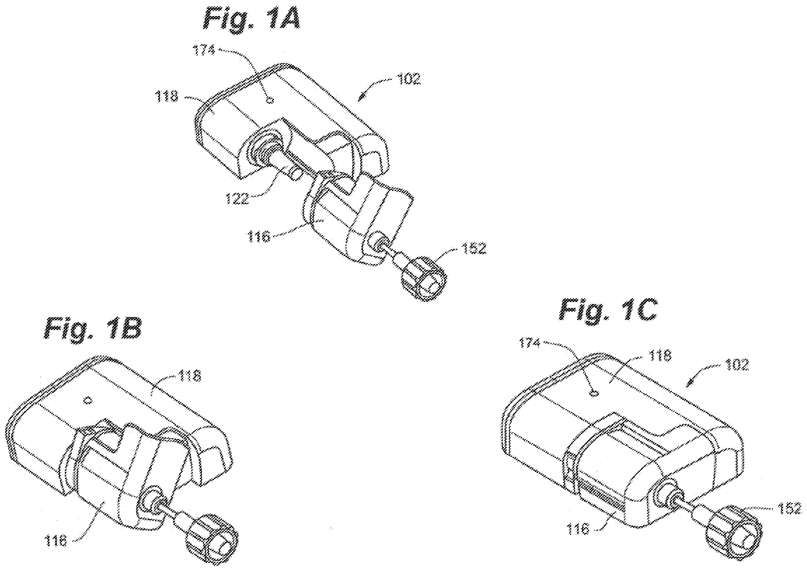

Referring to Figs. 1A-1C, a pump system 100 including a pump 102 is depicted in accordance with an embodiment of the disclosure. Drive unit 118 of pump 102 includes a drive mechanism 122 that mates with a recess in medicament cartridge 116 of pump 102 to attach the medicament cartridge 116 to the drive unit 118. Further details regarding example embodiments of such mechanisms can be found in U.S. Patent Publication No. 2017/0049957.

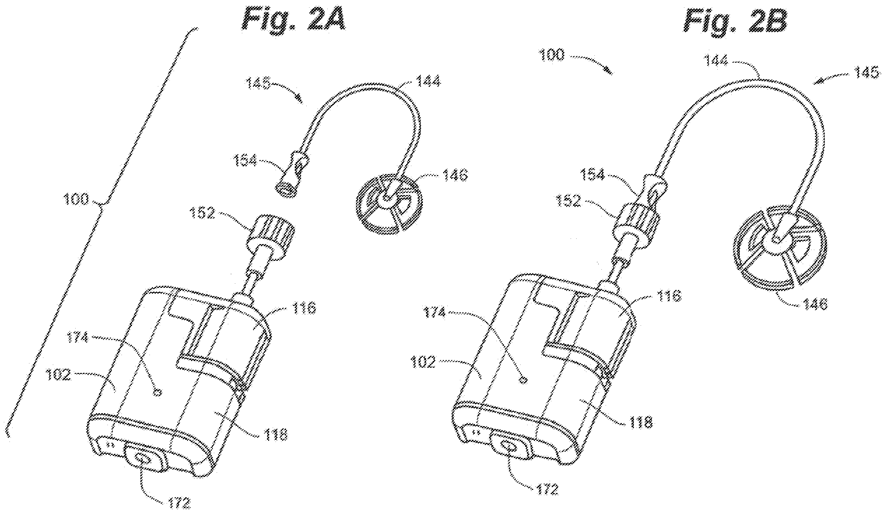

As depicted in the embodiment of Figs. 2A-2B, pump system 100 can include a pump 102 and an infusion set 145. Figure 2B depicts infusion set 145 connected to pump 102 via connectors 154 and 152. Infusion set 145 can include tubing 144 extending between a connector 154 and a site connector 146. Site connector 146 can be configured to be attached to an infusion site on a user, while pump 102 can be carried in a separate location, such as the user's pocket or another location on the user's body. Various lengths of tubing 144 can be used in this embodiment to accommodate the user's preference. Further details regarding such pumps can be found in U.S. Patent Nos. 9,993,595; 10,279,106; and 10,279,107.

In one embodiment, pump 102 includes a processor that controls operations of the pump and, in some embodiments, may communicate in either one-way or two-way modes to, e.g., receive operational commands and/or other signals, including data, from a separate device and/or, e.g., to send signals, including data, to a separate device. Pump 102 can include one or more buttons configured to cause the processor to initiate one or more functions. In the depicted embodiment, pump 102 includes only a single button 172, although more than one button may be present on pump 102. Button 172 can be configured to, for example, initiate delivery of medicament. Any single button such as button 172 can be utilized to execute a plurality of functions or operations. For example, a single press of button may initiate one function, holding the button down for a predetermined period of time may initiate another function, etc. Because the depicted pump 102 optionally does not itself include a display or user interface, information and feedback regarding medicament delivery or dosing initiated with button 172 can be communicated to and displayed on a remote control device or other device having a display and/or other type of user interface. Further details regarding use of button 172 can be found in U.S. Patent Publication No. 2018/0193555.

In one embodiment, pump 102 includes a light source, such as a light emitting diode (LED) 174. Light source 174 can be configured to provide user feedback regarding user input and/or the performance of a desired function. For example, in one embodiment, light source 174 can illuminate or blink one or more times to indicate that the one or more buttons 172 have been activated and/or that a desired function has been initiated. In one embodiment, pump 102 can additionally and/or alternatively vibrate and/or provide audible notifications to indicate that the one or more buttons 172 have been activated and/or that a desired function has been initiated or, e.g., to provide user feedback regarding user input and/or the performance of the desired function. Illumination of light source 174 and/or vibrations and/or audible notifications may be executed in any number of patterns, frequencies, durations, sequences, combinations, colors, brightness levels, etc. to indicate particular information, such as particular input received and/or particular functions or operations enabled and/or initiated, to the pump user or caregiver. In other embodiments, pump 102 includes two indicator lights 174.

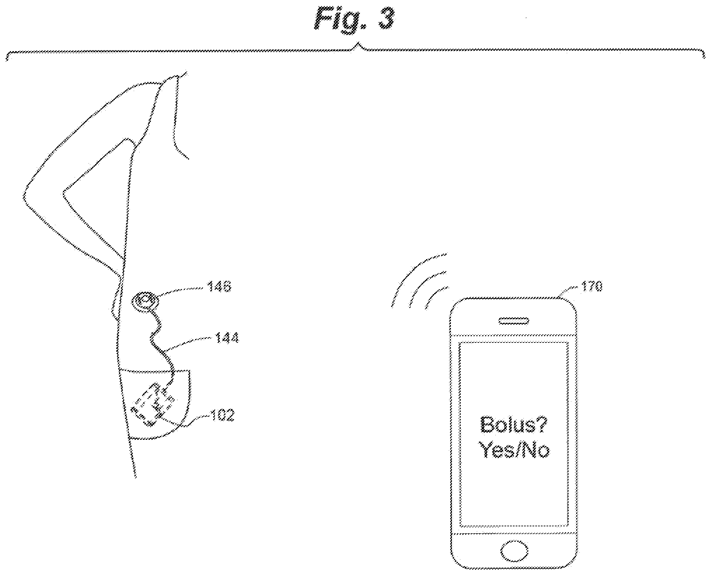

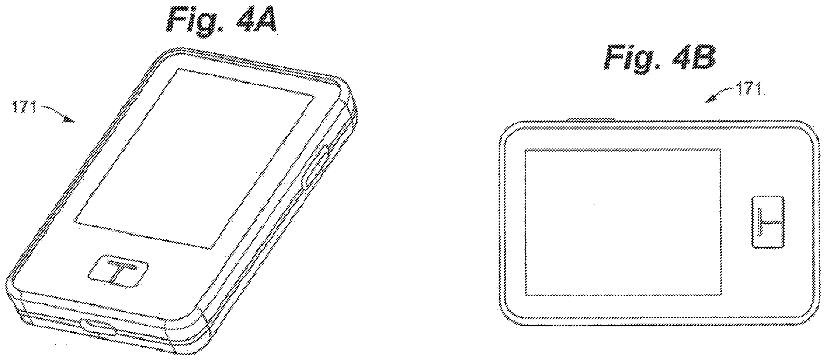

Referring to Figs. 3-4B, one or more remote control devices 170, 171 can be used to communicate with the processor of pump 102 to control delivery of medicament and transfer data with pump 102 via a wired or a wireless electromagnetic signal, such as via, e.g., a near field communication (NFC) radio frequency (RF) modality or other RF modalities such as Bluetooth^{®}, Bluetooth^{®} low energy, mobile or Wi-Fi communication protocols, for example, according to embodiments of the present disclosure. Such a remote control can include, for example, a mobile communication device 170, such as a smart phone (as depicted in Fig. 3) executing a software application for control of the pump, a dedicated remote controller 171 (as depicted in Figs. 4A-4B) designed for use with pump, a wearable electronic watch or electronic health or fitness monitor or personal digital assistant (PDA), etc., or a tablet, laptop or personal computer. Such communications between (and among) the one or more remote control devices 170, 171 and pump 102 may be one-way or two-way for, e.g., effective transfer of data among the devices and the pump, control of pump operations, updating software on the devices and/or pump, and allowing pump-related data to be viewed on the devices and/or pump.

Embodiments of the present invention include components capable of and methods using wired and wireless transmission and receipt of signals for exchange of information and commands between and among any of the components as described herein, including, e.g., between a pump and a smartphone; among a pump, a CGM and a smartphone; between a dedicated remote controller and a pump; among a dedicated remote controller, a CGM and a pump; among a dedicated remote controller, a BGM and a pump, and other combinations as would be contemplated by those of skill in the art.

As noted above, patch pumps such as pump 102 are typically worn directly on the user's body via an adhesive patch or can alternatively be carried near the body in, e.g., a pocket. Embodiments depicted and described herein provide a more versatile and secure way to hold and carry a user-wearable infusion pump. The figures discussed below depict and describe such a cradle/holder or carrying case for an infusion pump. Various features and advantages of such carriers include, for example, 1) an open ended cradle design that allows for lateral insertion and removal of the pump from either end; 2) an open ended cradle design that allows for multiple set connector orientations; 3) an open ended cradle design that prevents infusion line snags during removal; 4) an open ended cradle design that allows for insertion after an infusion set has been attached to set connector; and 5) a flexible retaining sleeve that securely retains the pump in the holder while allowing for easy insertion and removal.

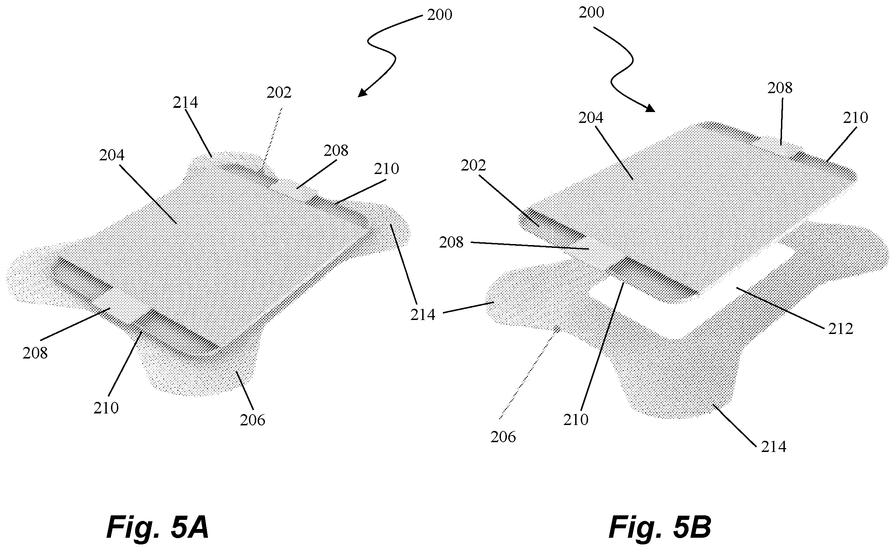

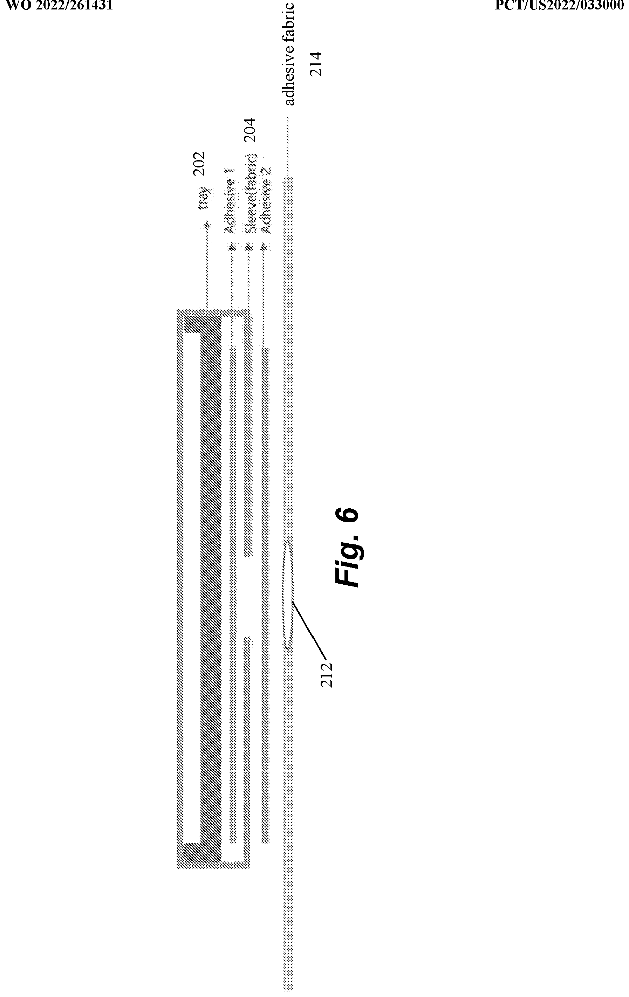

In particular, Figures 5A-5B and 6 depict a pump carrier or holder 200 according to an embodiment of the disclosure. Carrier 200 can generally include a tray 202, a sleeve 204 extending over the tray and an adhesive patch 206. Referring to Figure 6, sleeve 204 can extend around at least part of a bottom surface of tray 202 and be affixed thereto with an adhesive. The sleeve 204 and tray 202 can further be affixed to the adhesive patch 206 with an adhesive.

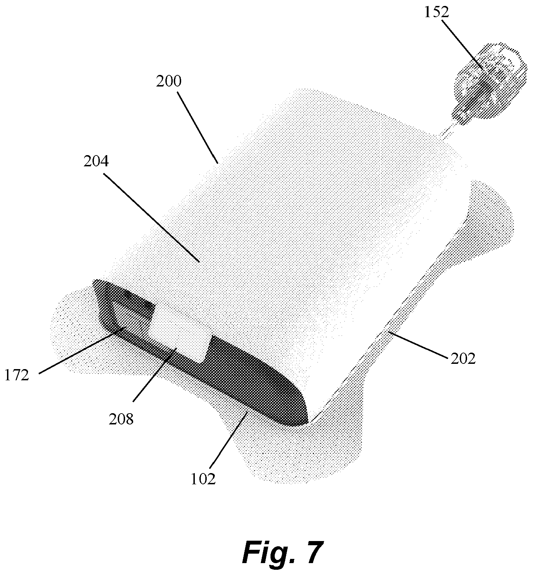

Sleeve 204 can be comprised of a flexible, woven fabric material having elastic properties such as, for example, spandex. As can be seen in Figure 6, sleeve 204 wraps around the longer sides of tray 202 and is adhered to the underside of tray leaving openings at both ends 210 of the tray 202. This advantageously enables a pump such as pump 102 to be inserted or removed from either end 210 of the tray 202 depending on the more convenient orientation for the user at a given time (See Figure 7). The elastic nature of sleeve 204 enables it to stretch to aid in inserting the pump onto tray 202 and then flex back towards its original shape to provide a force biasing the pump 102 onto tray 202 to securely hold the pump 102 on the tray 202. Sleeve 204 can further include one or more tabs 208 at either or both ends of sleeve 204 to aid in lifting of sleeve 204 for insertion and/or removal of pump from carrier 200. For example, by lifting a tab 208, the force of the sleeve 204 on the pump 102 can be alleviated to allow removal of pump 102 from carrier 200. In embodiments, tabs 208 can be thin, stiff, e.g., plastic, material (i.e., stiffer than the material of sleeve) bonded to sleeve 204 that provides a grasping point for user to lift and stretch the sleeve 204 to ease insertion and removal of pump 100. Providing a tab 208 adjacent each end 210 of tray 202 further aids in the versatility of the device to insert the pump 102 at either end 210 of the tray 202. Referring to Figure 7, in some embodiments tabs 208 can be offset from the location of a pump button 172 on pump 102 so as not to interfere with the use of button 172 when the pump 102 is in the carrier 200.

Patch 206 can include an adhesive on the bottom of patch for adhering to a user's body. In this embodiment, patch 206 includes a central aperture 212 beneath the tray 202. This reduces the amount of adhesive that will be applied to the user's skin to make it easier for the user to remove the carrier 200 from the body (while still providing sufficient adhesive to securely retain the carrier on the body). Patch 206 can have a shape that generally matches the shape of tray 202 along the sides and ends except for one or more protruding corners 214. Corners 214 can provide greater stability for pump 100 and carrier 200 by adhering to the user beyond a perimeter shape of the pump and carrier and can further aid in removal of the carrier 200 by providing a convenient point from which to begin peeling the patch 206 from the skin. As depicted in Figure 6, sleeve can be attached to tray and tray and sleeve attached to patch with an adhesive. In other embodiments, one or both attachments can be made using ultrasonic welding.

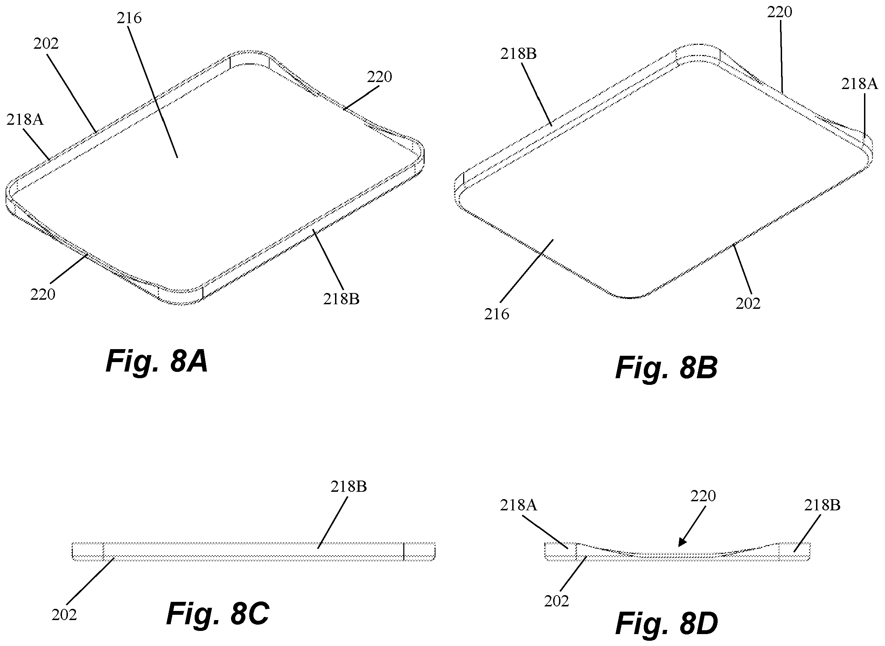

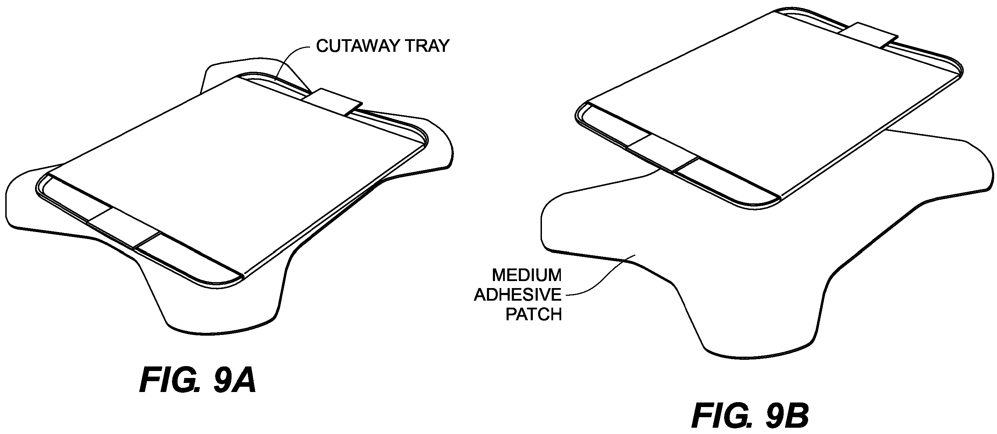

Referring now primarily to Figures 8A-8D, a tray 202 can have a tray body 216 having an outer perimeter matching a shape of a pump to be retained on tray. One or more upwardly extending retention walls 218A, 218B can extend around at least a portion of the outer perimeter of the tray body 216. In some embodiments, tray can include a single retention wall extending around the entire outer perimeter of tray body at a generally uniform height. In the depicted embodiment, a first retention wall 218A extends the full length of one side of tray 202 and a second retention wall 218B extends the full length of the opposite side of tray 202B. Each retention wall 218A, 218B further extends around the corners of the tray body 216 in order to firmly retain the pump at all four corners of the pump. The retention walls 218A, 218B can then taper down to tray body along the ends of tray body to form openings 220 between retention walls 218A, 218B at opposing ends of tray 202. Openings 220 can make it easier to insert pump into and remove pump from between tray 202 and sleeve. In other embodiments, retention walls may taper downwardly but provide a lower height wall between retention walls rather that tapering down to the tray body to provide additional retention while still providing openings. Figures 9A-9B depict a pump carrier 200 that incorporates the cutaway tray 202 of Figures 8A-8D. In the depicted embodiment, the tabs 208 of sleeve 204 generally align with and have approximately the same width as the openings 208 between retention walls 218A, 218B to provide additional support for retaining pump on tray.

The combination of sleeve 204 and tray 202 in the embodiments described herein therefore operates to securely retain a pump on the carrier while enabling easy insertion and removal of pump. Namely, sleeve 204 provides a force holding the pump on the tray to prevent the pump from falling vertically off of the tray whereas the one or more retention walls 218A, 218B of the tray prevent the pump from sliding horizontally out of the open ends of the sleeve 204. The elastic nature of sleeve 204 and the tabs 208 that aid lifting the sleeves enable easier insertion and removal of the pump by pulling the sleeve away from the pump without sacrificing secure retention of the pump when the sleeve flexes back towards its original shape to hold the pump on the tray.

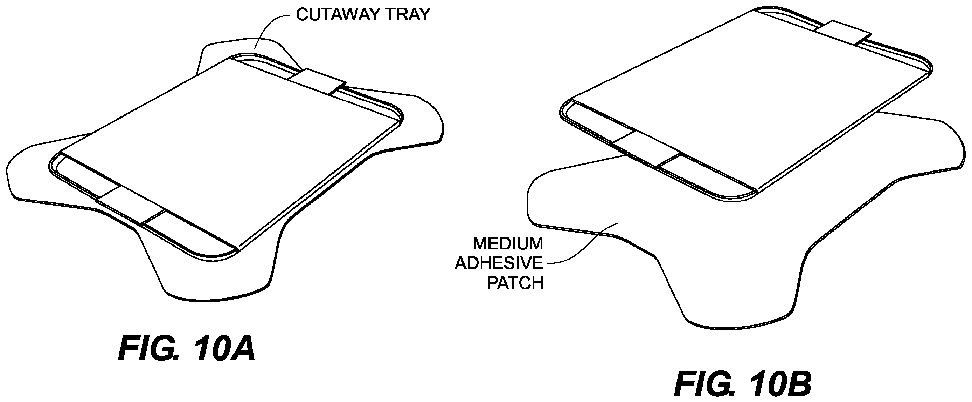

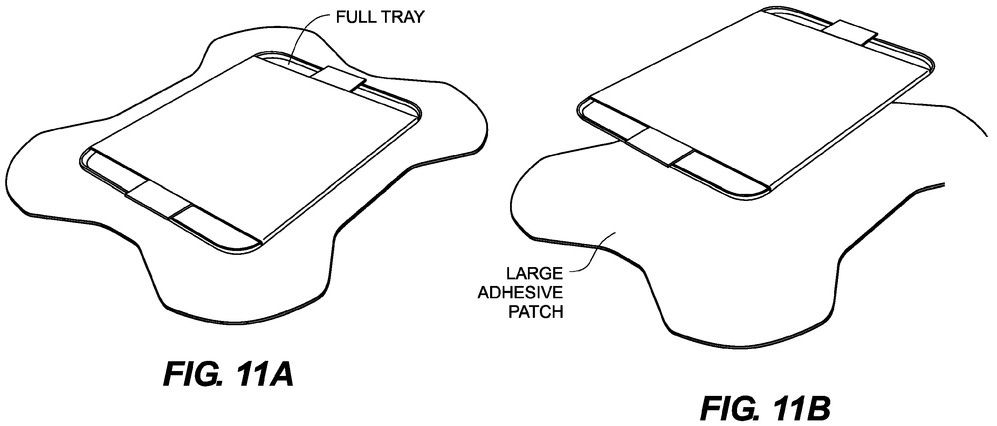

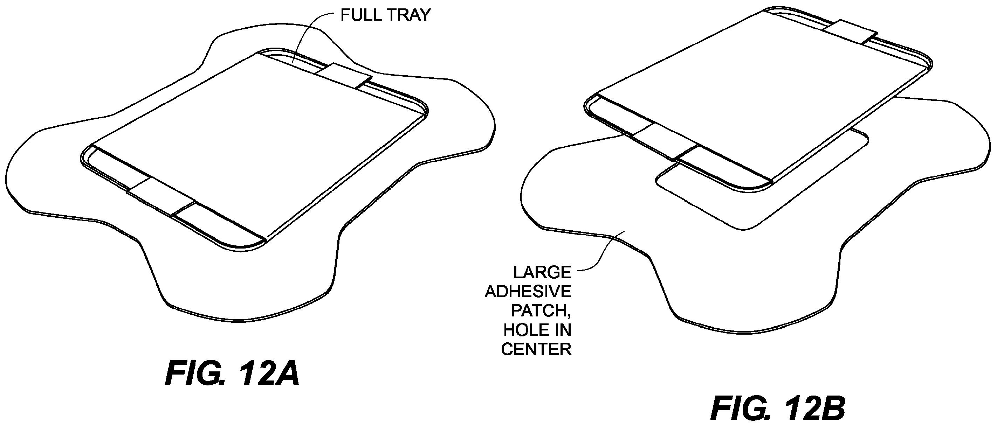

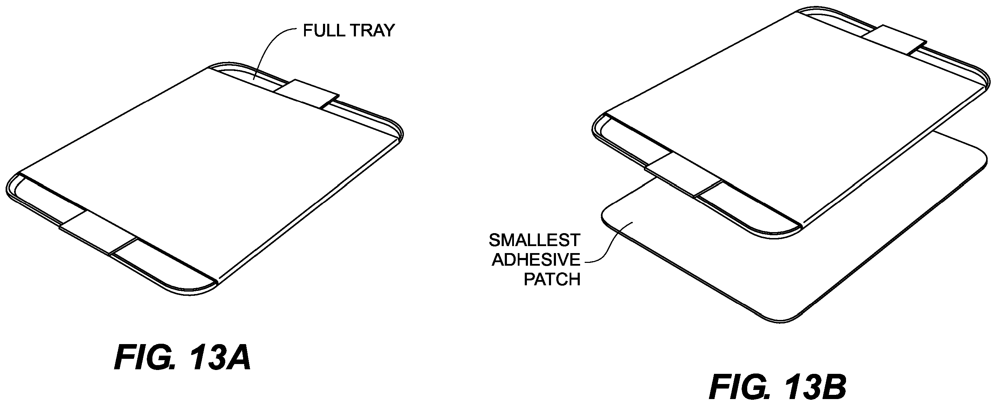

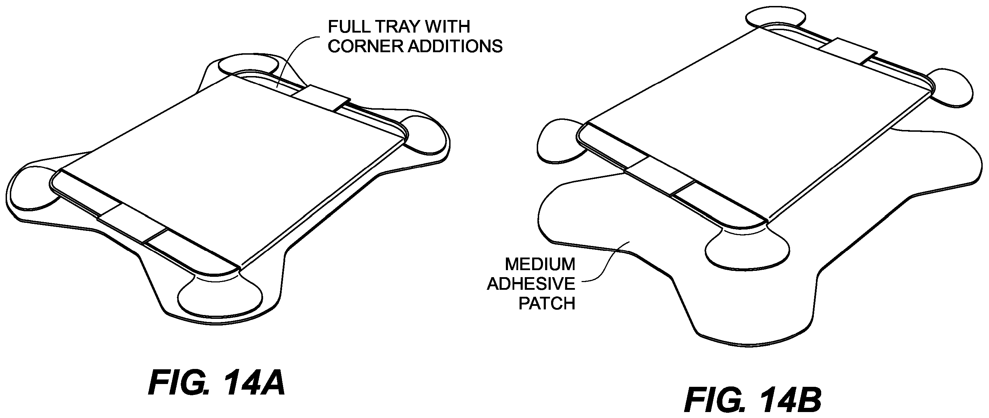

Figures 10A-10B depict another embodiment that is similar to pump carrier 200 that does not include the central aperture 212 that reduces the amount of adhesive interfacing with the user's skin. Figures 11A-11B and 12A-12B depict embodiments that employ a larger patch, with and without the central aperture. Figures 13A-13B depict an embodiment having a low profile adhesive patch having a perimeter size and shape generally matching that of the tray. Figures 14A-14B depict an embodiment having adhesive corners extending from tray that serve to retain the tray on the adhesive patch.

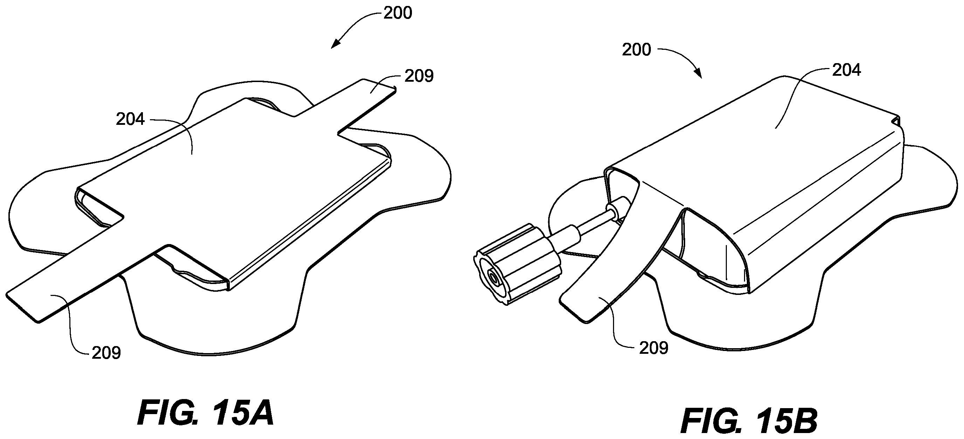

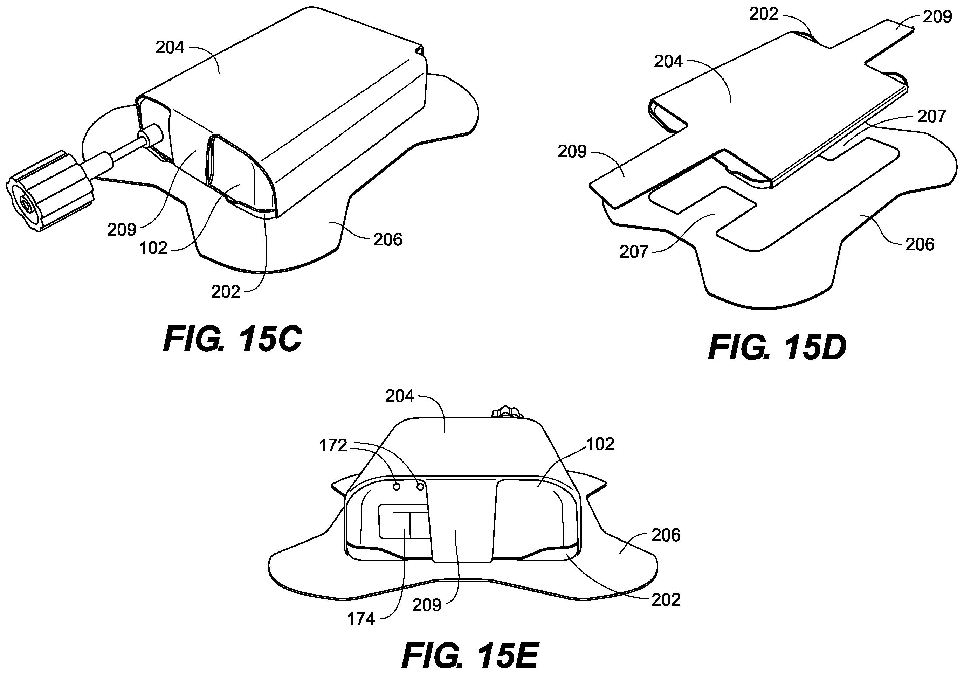

Figs. 15A-15E depict a holder or carrier 200 for a user-wearable infusion pump according to another embodiment. In this embodiment, carrier 200 includes an elongated tab 209 at each end of the sleeve 204 instead of the tabs 208 described above. In contrast to the relatively stiff, plastic material used for tab 208, elongated tab 209 can be comprised of a same or similar material as sleeve 204. In some embodiments, the elongated tabs 209 and sleeve 204 are unitarily formed as a monolithic construct from a single piece of material. Referring to Figures 15C and 15E, elongated tabs 209 can aid in retaining the pump 102 in the carrier 200 by wrapping around the pump 102 and being inserted beneath the tray 202 (i.e., between the tray 202 and the adhesive patch 206). As depicted in Figure 5D, in order to enable the elongated tabs 209 to be inserted into and removed from beneath the tray 202, portions 207 of the adhesive sleeve 206 beneath the tray 202 conforming to a perimeter shape of the tabs 209 are not provided with adhesive for adhering the tray 202 onto the adhesive patch 206. In some embodiments, silicon gel or other non-slip material can be disposed on a bottom surface of elongated tabs 209 to aid in retention and inhibit withdrawal of tabs 209 from beneath tray 209. Referring to Figure 15E, elongated tabs 209 can be sized and shaped such that indicator lights 174 of pump 102 are visible and bolus button 172 of pump 100 is accessible when the elongated tabs 209 are wrapped around pump 102 and disposed beneath tray 202.

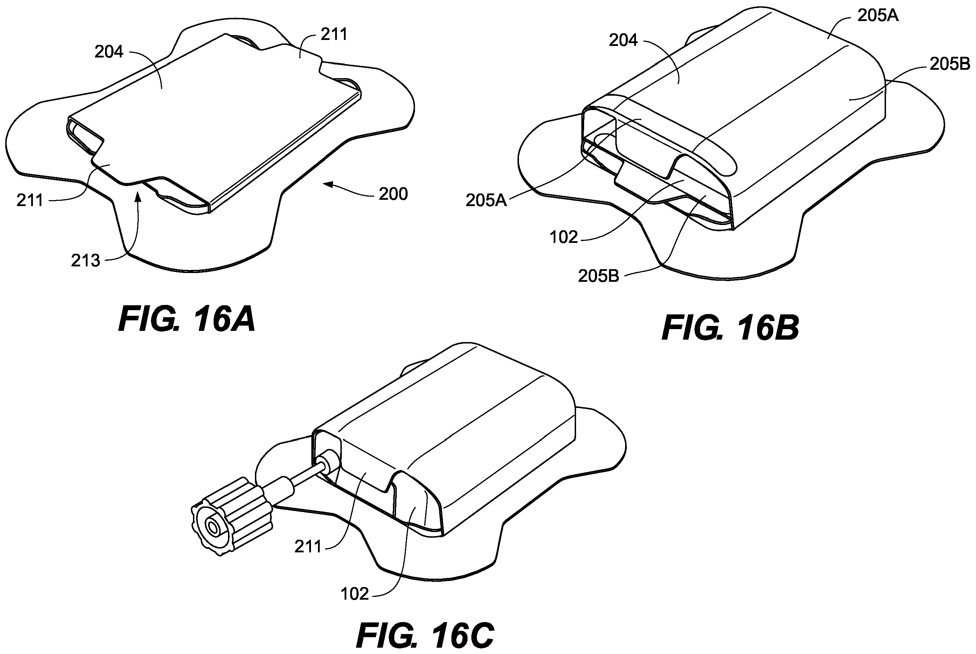

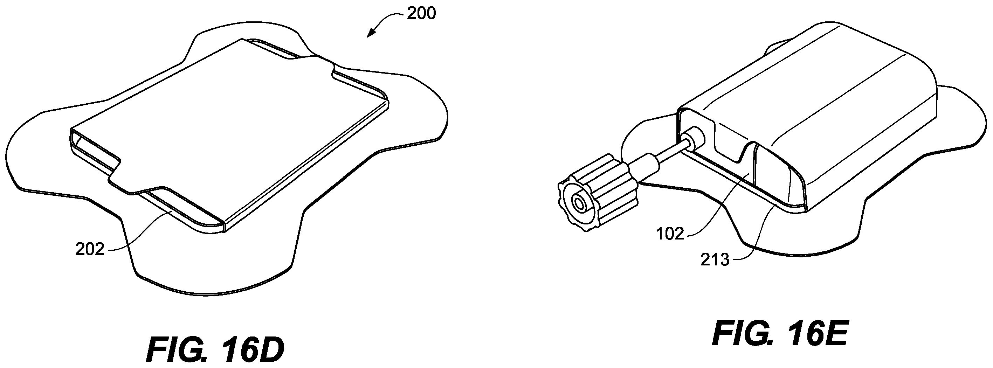

Figures 16A-16C depict another embodiment of a holder or carrier 200 for a user-wearable infusion pump. This embodiment also includes tabs 211 made of a softer material than tabs 208 and that, in some embodiments, can be comprised of the same material of and unitarily formed with sleeve 204. Sleeve 204 can include silicon or other non-slip material printed on one or more non-slip zones 205 on the inside of sleeve (note that Figure 16B depicts sleeve 204 with non-slip zones 205A with and 205B without a pump 102 inserted). In the depicted embodiment, non-slip zones 205 are provided on opposing outer ends of sleeve 204. Tabs 211 can alternatively or additionally have silicon or other non-slip material imprinted on the underside 213 of tabs. Non-slip printed areas 205, 213 can interface with pump 102 to enhance retention of pump 102 in carrier. Figures 16D-16E depict a similar embodiment in which the retention wall 218 of the tray 202 extends around the full circumference of tray to provide further resistance against the pump 102 inadvertently slipping out of the sleeve 204.

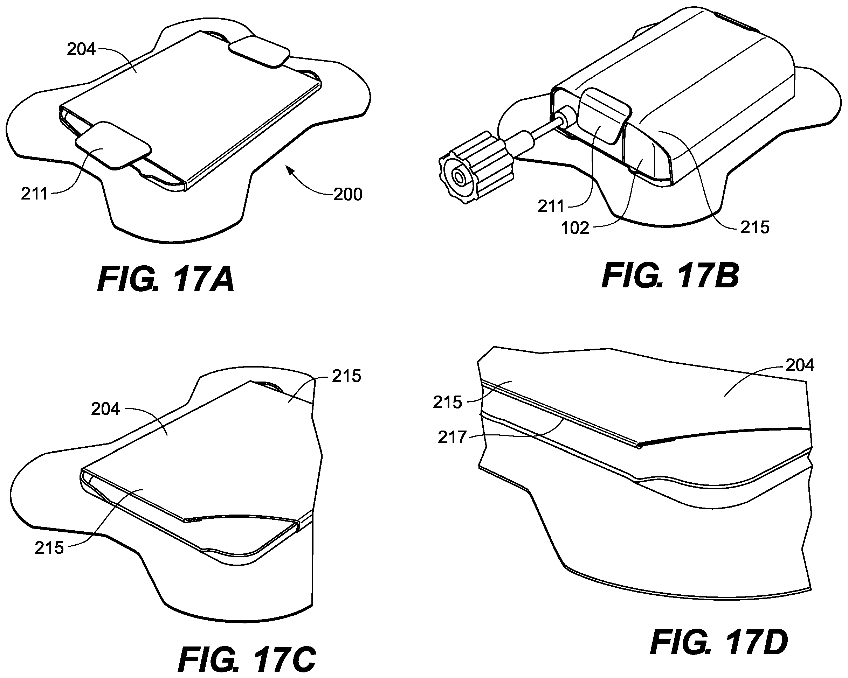

Figures 17A-17C depict a further embodiment of a holder or carrier 200 for a user-wearable infusion pump. In this embodiment, a hem 215 is created along each end of the sleeve 204. The hems 215 bolster the strength of the sleeve 204 to aid in retention of the pump 102 by effectively doubling the thickness of the sleeve 204 at the location of the hems 205. In the depicted embodiment, soft tabs 211 have been attached to the sleeve 204 due to the hem 215 complicating formation of unitary tabs such as those described above. Figure 17D depicts a similar embodiment that further includes an elastic edge 217 affixed to the hem 215 at the ends of the sleeve to further enhance pump retention.

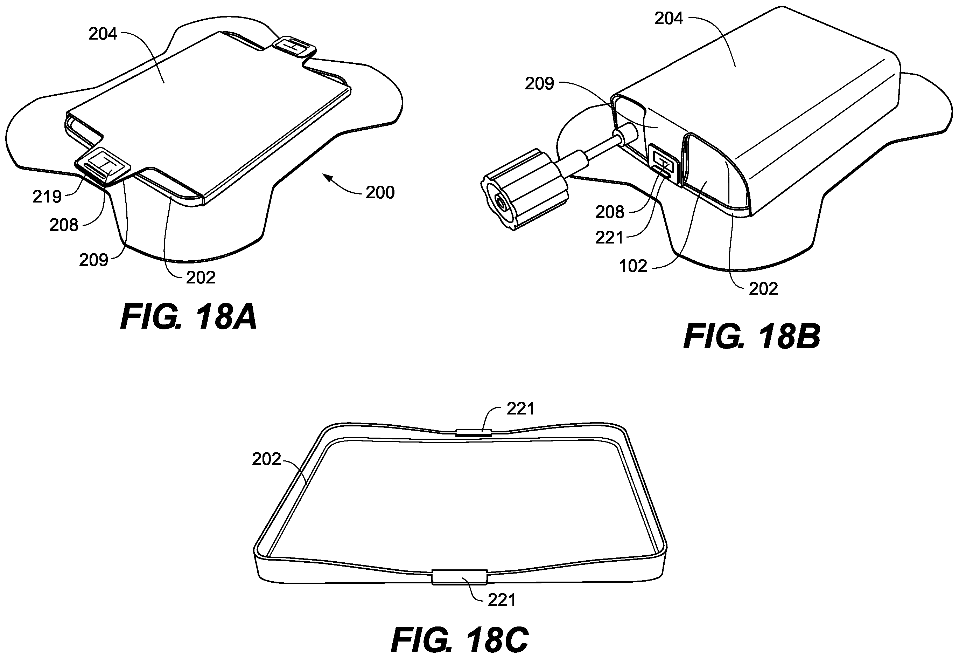

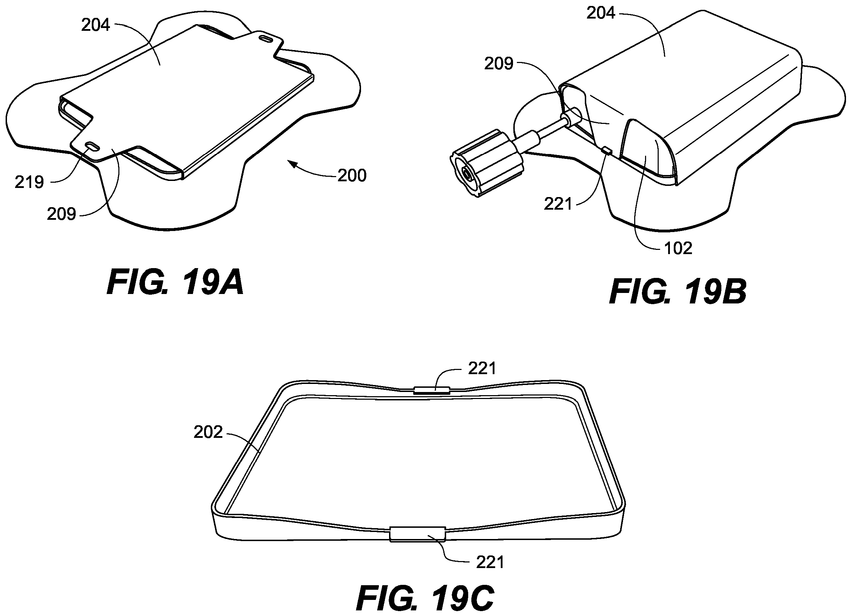

Another embodiment of a holder or carrier 200 for a user-wearable infusion pump is depicted in Figures 18A-18C that enables a user to selectively lock the sleeve 204 over the pump 102. In this embodiment, sleeve 204 includes unitary soft tabs 209 with tab 208 comprised of a more rigid material, e.g., plastic, affixed thereto. Tabs 208, 209 include aligned slots 219 that interface with a rigid tab 221 projecting outwardly from each end of tray 202 to selectively lock sleeve 204 over pump 102. The rigid material of tab 208 enhances the ability of the tabs 208, 209 of the sleeve to be retained on tray tab 221 until removed by the user. This effectively prevents the pump 102 from being removed from the sleeve 204 until the sleeve tabs 208, 209 are unhooked from the tray tab 221. Figures 19A-19C depict a similar embodiment that does not include hard tabs 208 such that only soft tabs 209 are inserted and retained on tray tabs 221 of tray 202.

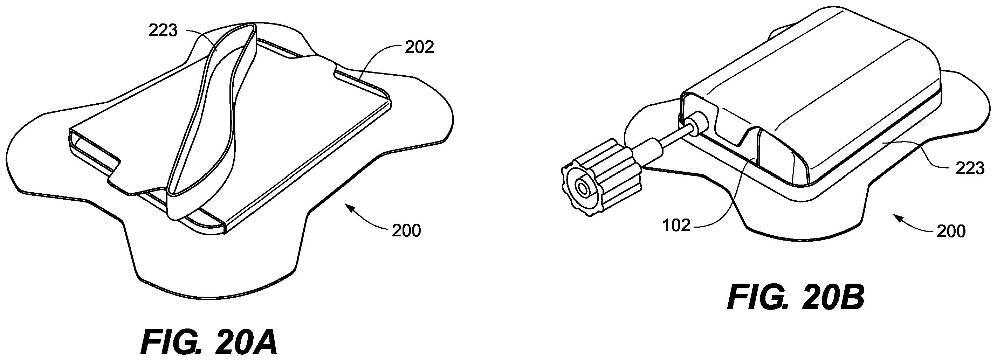

Figures 20A-20B depict a holder 200 for a user-wearable infusion pump according to a further embodiment. This embodiment depicts an additional elastic band 223 that can be employed with any of the embodiments disclosed herein. As depicted in Figure 20B, elastic band 223 can be selectively disposed around the tray 202 by stretching the band 223 around the tray 202 and then releasing the band 223 to allow the elastic nature to firmly retain the band 223 around the tray 202. The elastic band 223 can at least partially overlap the pump 102 in order to further enhance retention of the pump on the carrier. In a further embodiment, the band 223 of Figures 20A-20B can be combined with the tab features 208, 209, 221 of Figures 18A-18C and 19A-19C. In such embodiments, the band 223 can be attached to tabs 208 and/or 209 and when those tabs are pulled down and attached to the tray tabs 221 the band 223 is pulled with the tabs 208, 209 and automatically positioned around the tray 202 and pump 102. In additional to pump retention, elastic band 223 in this embodiment can further serve to constrain a long infusion set that gets wrapped around the pump prior to positioning the band 223 around the pump.

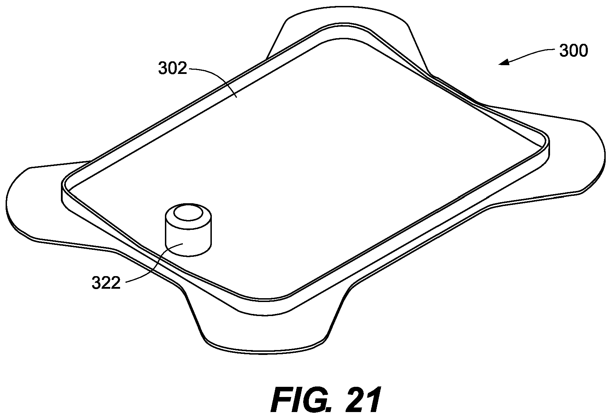

In some embodiments, a patch pump carried by one or more of the tray embodiments disclosed herein includes a set connector 152 that extends from pump 102 and carrier 200 for connection to an infusion set 145 that includes a site connector 146 that attaches to the user at a separate location from the carrier 200 for delivery of insulin or other medicament through a cannula inserted into the user's skin. In particular, such pump holders can be advantageous for use with shorter length (e.g., 5 inch) infusion sets that do not provide sufficient length to store a pump in a user's pocket or otherwise off of the body. In other embodiments, a cannula can be integrated with the carrier such that the pump delivers medicament to the user at a location directly beneath the carrier. For example, Figure 21 depicts a pump carrier 300 including a tray 302 having a connector 322 defined in the tray. Connector 322 can include a passage through tray body through which a needle can be advanced to insert a cannula into the skin of the patient directly below the tray 302. In such an embodiment, a pump inserted into carrier can include an external feature that interfaces with the connector 322 to deliver medicament from the pump through the connector 322 and into the body through the cannula. Further details regarding pump carriers having integrated connectors for delivering medicament directly below the carriers and corresponding infusion pumps can be found in U.S. Patent No. 10,279,106.

In embodiments, a user-wearable infusion pump system includes a user-wearable infusion pump configured to contain a medicament and a retention frame including a frame base having a first side and an opposing second side and a first end and opposing second end defining a frame perimeter having a shape generally matching an outer perimeter of the user-wearable pump. An elastic sleeve can be attached to the retention frame and extend across the retention frame from the first side of the retention frame to the second side of the retention frame. The elastic sleeve can be configured to be stretched away from the retention frame to enable insertion of the user-wearable infusion pump onto the retention frame and to flex back against the user-wearable infusion pump to provide a force biasing the infusion pump on the retention frame.

In some embodiments, the elastic sleeve provides an opening with the retention frame at both the first and the second end of the retention frame such that the user-wearable infusion pump can be inserted into the retention frame from either the first end or the second end of the retention frame.

In some embodiments, the elastic sleeve extends around the first side and the second side of the retention frame such that the elastic sleeve is attached to the retention frame on a bottom surface of the retention frame adjacent both the first side and the second side of the retention frame.

In some embodiments, the system includes a tab extending outwardly from the elastic sleeve adjacent at least one of the first end and the second end of the retention frame.

In some embodiments, there is a tab extending outwardly from the elastic sleeve adjacent both the first end and the second end of the retention frame.

In some embodiments, the tab comprises a more rigid material than the elastic sleeve.

In some embodiments, the tab comprises a flexible material configured to be releasably wrapped around an end of the user-wearable infusion pump.

In some embodiments, the user-wearable infusion pump includes at least one of a button and one or more indicator lights, and the tab is offset from the at least one of the button and the one or more indicator lights such that the button is accessible and/or the one or more indicator lights are visible when the user-wearable infusion pump is disposed on the retention frame.

In some embodiments, the retention frame further includes a retention wall extending upwardly from and around the frame perimeter.

In some embodiments, the retention frame includes a first retention wall extending along the first side of the retention frame and around corners at the first end and the second end of the retention frame adjacent the first side and a second retention wall extending along the second side of the retention frame and around corners at the first end and the second end of the retention frame adjacent the second side. The first retention wall and the second retention wall can define openings therebetween at both the first end and the second end of the retention frame.

In embodiments, a pump holder for selectively retaining a user-wearable infusion pump, comprising can include a retention frame including a frame base having a first side and an opposing second side and a first end and opposing second end defining a frame perimeter having a shape generally matching an outer perimeter of a user-wearable pump and an elastic sleeve attached to the retention frame and extending across the retention frame from the first side of the retention frame to the second side of the retention frame. The elastic sleeve can be configured to be stretched away from the retention frame to enable insertion of the user-wearable infusion pump onto the retention frame and to flex back against the user-wearable infusion pump to provide a force biasing the infusion pump on the retention frame.

In some embodiments, the elastic sleeve provides an opening with the retention frame at both the first and the second end of the retention frame such that the user-wearable infusion pump can be inserted into the retention frame from either the first end or the second end of the retention frame.

In some embodiments, the elastic sleeve extends around the first side and the second side of the retention frame such that the elastic sleeve is attached to the retention frame on a bottom surface of the retention frame adjacent both the first side and the second side of the retention frame.

In some embodiments, a tab can extend outwardly from the elastic sleeve adjacent at least one of the first end and the second end of the retention frame.

In some embodiments, there is a tab extending outwardly from the elastic sleeve adjacent both the first end and the second end of the retention frame.

In some embodiments, the tab comprises a more rigid material than the elastic sleeve.

In some embodiments, the tab comprises a flexible material configured to be releasably wrapped around an end of the user-wearable infusion pump.

In some embodiments, the tab is configured to be offset from at least one of a button of the user-wearable pump and one or more indicator lights of the user-wearable pump such that the button is accessible and/or the one or more indicator lights are visible when the user-wearable infusion pump is disposed on the retention frame.

In some embodiments, the retention frame further includes a retention wall extending upwardly from and around the frame perimeter.

In some embodiments, the retention frame includes a first retention wall extending along the first side of the retention frame and around corners at the first end and the second end of the retention frame adjacent the first side and a second retention wall extending along the second side of the retention frame and around corners at the first end and the second end of the retention frame adjacent the second side. The first retention wall and the second retention wall can define openings therebetween at both the first end and the second end of the retention frame.

Although embodiments described herein may be discussed in the context of the controlled delivery of insulin, delivery of other medicaments, singly or in combination with one another or with insulin, including, for example, glucagon, pramlintide, etc., as well as other applications are also contemplated. Device and method embodiments discussed herein may be used for pain medication, chemotherapy, iron chelation, immunoglobulin treatment, dextrose or saline IV delivery, treatment of various conditions including, e.g., pulmonary hypertension, or any other suitable indication or application. Non-medical applications are also contemplated.

With regard to the above detailed description, like reference numerals used therein may refer to like elements that may have the same or similar dimensions, materials, and configurations. While particular forms of embodiments have been illustrated and described, it will be apparent that various modifications can be made without departing from the scope of the embodiments herein. Accordingly, it is not intended that the invention be limited by the forgoing detailed description.

Citation of the above patents, patent applications, publications and documents is not an admission that any of the foregoing is pertinent prior art, nor does it constitute any admission as to the contents or date of these documents.

Modifications may be made to the foregoing embodiments without departing from the basic aspects of the technology. Although the technology may have been described in substantial detail with reference to one or more specific embodiments, changes may be made to the embodiments specifically disclosed in this application, yet these modifications and improvements are within the scope of the technology. The technology illustratively described herein may suitably be practiced in the absence of any element(s) not specifically disclosed herein. The terms and expressions which have been employed are used as terms of description and not of limitation and use of such terms and expressions do not exclude any of the features shown and described or portions thereof and various modifications are possible within the scope of the technology claimed. Although the present technology has been specifically disclosed by representative embodiments and optional features, modification and variation of the concepts herein disclosed may be made, and such modifications and variations may be considered within the scope of this technology.

## Claims

1. A pump holder (200; 300) for selectively retaining a user-wearable infusion pump, comprising:
a retention frame (202; 302) including a frame base having a first side and an opposing second side and a first end (210) and opposing second end (210) extending between the first side and the second side, the first side, second side, first end, and second end defining a frame perimeter having a shape generally matching an outer perimeter of a user-wearable pump, the retention frame further including one or more retention walls extending vertically from the frame perimeter; and
an elastic sleeve (204) attached to the retention frame and extending across the retention frame from the first side of the retention frame to the second side of the retention frame, wherein the elastic sleeve is configured to be stretched away from the retention frame to enable insertion of the user-wearable infusion pump onto the retention frame and to flex back against the user-wearable infusion pump to provide a force biasing the infusion pump on the retention frame; and
an adhesive patch (206) configured to adhere the pump holder to a user.

2. The pump holder of claim 1, wherein the elastic sleeve provides an opening with the retention frame at both the first and the second end of the retention frame such that the user-wearable infusion pump can be inserted into the retention frame from either the first end or the second end of the retention frame.

3. The pump holder of claim 1 or claim 2, wherein the elastic sleeve extends around the first side and the second side of the retention frame such that the elastic sleeve is attached to the retention frame on a bottom surface of the retention frame adjacent both the first side and the second side of the retention frame.

4. The pump holder of any of claims 1-3, further comprising a tab (208; 209; 211) extending outwardly from the elastic sleeve adjacent at least one of the first end and the second end of the retention frame.

5. The pump holder of claim 4, wherein there is a tab extending outwardly from the elastic sleeve adjacent both the first end and the second end of the retention frame.

6. The pump holder of claim 4 or claim 5, wherein the tab (208) comprises a more rigid material than the elastic sleeve.

7. The pump holder of claim 4, wherein the tab (209) comprises a flexible material configured to be releasably wrapped around an end of the user-wearable infusion pump.

8. The pump holder of any of claims 4-7, wherein the tab is configured to be offset from at least one of a button of the user-wearable pump and one or more indicator lights of the user-wearable pump such that the button is accessible and/or the one or more indicator lights are visible when the user-wearable infusion pump is disposed on the retention frame.

9. The pump holder of any of claims 1-8, wherein the one or more retention walls comprises a retention wall (218; 218A; 218B) extending upwardly from and around the frame perimeter.

10. The pump holder of any of claims 1-8, wherein the one or more retention walls comprise a first retention wall (218A) extending along the first side of the retention frame and around corners at the first end and the second end of the retention frame adjacent the first side and a second retention wall (218B) extending along the second side of the retention frame and around corners at the first end and the second end of the retention frame adjacent the second side, and wherein the first retention wall and the second retention wall define openings (220) therebetween at both the first end and the second end of the retention frame.

11. The pump holder of any of claims 1-10, further comprising an elastic band (223) configured to be selectively disposed around the frame perimeter.

12. The pump holder of any of claims 1-11, wherein the retention frame further comprises a connector (322) defining a passage through retention frame configured to receive a cannula.

13. A user-wearable infusion pump system, comprising:
a user-wearable infusion pump configured to contain a medicament; and
the pump holder of any of claims 1-12.

## Patentansprüche

1. Pumpenhalter (200; 300) zum selektiven Halten einer vom Benutzer tragbaren Infusionspumpe, umfassend:
einen Halterahmen (202; 302), der eine Rahmenbasis, die eine erste Seite und eine gegenüberliegende zweite Seite und ein erstes Ende (210) und ein gegenüberliegendes zweites Ende (210) aufweist, die sich zwischen der ersten Seite und der zweiten Seite erstrecken, beinhaltet, wobei die erste Seite, die zweite Seite, das erste Ende und das zweite Ende einen Rahmenumfang definieren, der eine Form aufweist, die im Allgemeinen mit einem Außenumfang einer vom Benutzer tragbaren Pumpe übereinstimmt, wobei der Halterahmen ferner eine oder mehrere Haltewände umfasst, die sich vertikal von dem Rahmenumfang erstrecken; und
eine elastische Hülse (204), die an dem Halterahmen angebracht ist und sich von der ersten Seite des Halterahmens zu der zweiten Seite des Halterahmens über den Halterahmen erstreckt, wobei die elastische Hülse dazu konfiguriert ist, von dem Halterahmen weg gedehnt zu werden, um das Einführen der vom Benutzer tragbaren Infusionspumpe auf den Halterahmen zu ermöglichen, und sich gegen die vom Benutzer tragbare Infusionspumpe zu biegen, um eine Kraft bereitzustellen, die die Infusionspumpe auf den Halterahmen vorspannt; und
ein Klebepflaster (206), das dazu konfiguriert ist, den Pumpenhalter an einen Benutzer zu kleben.

2. Pumpenhalter nach Anspruch 1, wobei die elastische Hülse eine Öffnung mit dem Halterahmen sowohl an dem ersten als auch dem zweiten Ende des Halterahmens derart bereitstellt, dass die vom Benutzer tragbare Infusionspumpe entweder von dem ersten Ende oder dem zweiten Ende des Halterahmens in den Halterahmen eingeführt werden kann.

3. Pumpenhalter nach Anspruch 1 oder Anspruch 2, wobei sich die elastische Hülse um die erste Seite und die zweite Seite des Halterahmens derart erstreckt, dass die elastische Hülse an dem Halterahmen an einer unteren Fläche des Halterahmens benachbart sowohl zu der ersten Seite als auch zu der zweiten Seite des Halterahmens angebracht ist.

4. Pumpenhalter nach einem der Ansprüche 1 bis 3, ferner umfassend eine Lasche (208; 209; 211), die sich von der elastischen Hülse benachbart zu mindestens einem von dem ersten Ende und dem zweiten Ende des Halterahmens nach außen erstreckt.

5. Pumpenhalter nach Anspruch 4, wobei eine Lasche vorhanden ist, die sich von der elastischen Hülse benachbart sowohl zu dem ersten Ende als auch zu dem zweiten Ende des Halterahmens nach außen erstreckt.

6. Pumpenhalter nach Anspruch 4 oder Anspruch 5, wobei die Lasche (208) ein steiferes Material als die elastische Hülse umfasst.

7. Pumpenhalter nach Anspruch 4, wobei die Lasche (209) ein flexibles Material umfasst, das dazu konfiguriert ist, lösbar um ein Ende der vom Benutzer tragbaren Infusionspumpe gewickelt zu werden.

8. Pumpenhalter nach einem der Ansprüche 4 bis 7, wobei die Lasche dazu konfiguriert ist, von mindestens einem von einem Knopf der vom Benutzer tragbaren Pumpe und einer oder mehreren Anzeigeleuchten der vom Benutzer tragbaren Pumpe derart versetzt zu sein, dass auf den Knopf zugegriffen werden kann und/oder die eine oder mehreren Anzeigeleuchten sichtbar sind, wenn die vom Benutzer tragbare Infusionspumpe an dem Halterahmen angeordnet ist.

9. Pumpenhalter nach einem der Ansprüche 1 bis 8, wobei die eine oder mehreren Haltewände eine Haltewand (218; 218A; 218B) umfassen, die sich von dem und um den Rahmenumfang nach oben erstreckt.

10. Pumpenhalter nach einem der Ansprüche 1 bis 8, wobei die eine oder mehreren Haltewände eine erste Haltewand (218A), die sich entlang der ersten Seite des Halterahmens und um Ecken an dem ersten Ende und dem zweiten Ende des Halterahmens benachbart zu der ersten Seite erstreckt, und eine zweite Haltewand (218B), die sich entlang der zweiten Seite des Halterahmens und um Ecken an dem ersten Ende und dem zweiten Ende des Halterahmens benachbart zu der zweiten Seite erstreckt, umfassen, und wobei die erste Haltewand und die zweite Haltewand Öffnungen (220) dazwischen sowohl an dem ersten Ende als auch dem zweiten Ende des Halterahmens definieren.

11. Pumpenhalter nach einem der Ansprüche 1 bis 10, ferner umfassend ein elastisches Band (223), das dazu konfiguriert ist, selektiv um den Rahmenumfang angeordnet zu werden.

12. Pumpenhalter nach einem der Ansprüche 1 bis 11, wobei der Halterahmen ferner einen Verbinder (322) umfasst, der einen Durchgang durch den Halterahmen definiert, der dazu konfiguriert ist, eine Kanüle aufzunehmen.

13. Vom Benutzer tragbares Infusionspumpensystem, umfassend:
eine vom Benutzer tragbare Infusionspumpe, die dazu konfiguriert ist, ein Medikament zu enthalten; und
den Pumpenhalter nach einem der Ansprüche 1 bis 12.

## Revendications

1. Support de pompe (200 ; 300) destiné à retenir de manière sélective une pompe à perfusion pouvant être portée par l'utilisateur, comprenant :
un cadre de retenue (202 ; 302) comprenant une base de cadre ayant un premier côté et un second côté opposé et une première extrémité (210) et une seconde extrémité opposée (210) s'étendant entre le premier côté et le second côté, le premier côté, le second côté, la première extrémité et la seconde extrémité définissant un périmètre de cadre ayant une forme correspondant généralement à un périmètre extérieur d'une pompe pouvant être portée par l'utilisateur, le cadre de retenue comprenant en outre une ou plusieurs parois de retenue s'étendant verticalement à partir du périmètre de cadre ; et
un manchon élastique (204) fixé au cadre de retenue et s'étendant en travers du cadre de retenue du premier côté du cadre de retenue au second côté du cadre de retenue, ledit manchon élastique étant configuré pour être étiré dans la direction opposée au cadre de retenue pour permettre l'insertion de la pompe à perfusion pouvant être portée par l'utilisateur sur le cadre de retenue et pour revenir contre la pompe à perfusion pouvant être portée par l'utilisateur pour fournir une force sollicitant la pompe à perfusion sur le cadre de retenue ; et
un patch adhésif (206) configuré pour coller le support de pompe sur un utilisateur.

2. Support de pompe de la revendication 1, dans lequel le manchon élastique fournit une ouverture avec le cadre de retenue à la fois au niveau de la première et de la seconde extrémité du cadre de retenue de sorte que la pompe à perfusion pouvant être portée par l'utilisateur peut être insérée dans le cadre de retenue à partir de la première extrémité ou de la seconde extrémité du cadre de retenue.

3. Support de pompe de la revendication 1 ou de la revendication 2, dans lequel le manchon élastique s'étend autour du premier côté et du second côté du cadre de retenue de sorte que le manchon élastique est fixé au cadre de retenue sur une surface inférieure du cadre de retenue adjacente à la fois au premier côté et au second côté du cadre de retenue.

4. Support de pompe de l'une quelconque des revendications 1 à 3, comprenant en outre une languette (208 ; 209 ; 211) s'étendant vers l'extérieur à partir du manchon élastique adjacente à au moins l'une de la première extrémité et de la seconde extrémité du cadre de retenue.

5. Support de pompe de la revendication 4, dans lequel une languette s'étend vers l'extérieur à partir du manchon élastique adjacente à la fois à la première extrémité et à la seconde extrémité du cadre de retenue.

6. Support de pompe de la revendication 4 ou de la revendication 5, dans lequel la languette (208) comprend un matériau plus rigide que le manchon élastique.

7. Support de pompe de la revendication 4, dans lequel la languette (209) comprend un matériau souple configuré pour être enroulé de manière amovible autour d'une extrémité de la pompe à perfusion pouvant être portée par l'utilisateur.

8. Support de pompe de l'une quelconque des revendications 4 à 7, dans lequel la languette est configurée pour être décalée par rapport à au moins un bouton de la pompe pouvant être portée par l'utilisateur et un ou plusieurs voyants lumineux de la pompe pouvant être portée par l'utilisateur de sorte que le bouton est accessible et/ou le ou les voyants lumineux sont visibles lorsque la pompe à perfusion pouvant être portée par l'utilisateur est disposée sur le cadre de retenue.

9. Support de pompe de l'une quelconque des revendications 1 à 8, dans lequel la ou les parois de retenue comprennent une paroi de retenue (218 ; 218A ; 218B) s'étendant vers le haut à partir du périmètre du cadre et autour de celui-ci.

10. Support de pompe de l'une quelconque des revendications 1 à 8, dans lequel la ou les parois de retenue comprennent une première paroi de retenue (218A) s'étendant le long du premier côté du cadre de retenue et autour des coins au niveau de la première extrémité et de la seconde extrémité du cadre de retenue adjacente au premier côté et une seconde paroi de retenue (218B) s'étendant le long du second côté du cadre de retenue et autour des coins au niveau de la première extrémité et de la seconde extrémité du cadre de retenue adjacente au second côté, et ladite première paroi de retenue et ladite seconde paroi de retenue définissant des ouvertures (220) entre elles à la fois au niveau de la première extrémité et de la seconde extrémité du cadre de retenue.

11. Support de pompe de l'une quelconque des revendications 1 à 10, comprenant en outre une bande élastique (223) configurée pour être disposée de manière sélective autour du périmètre du cadre.

12. Support de pompe de l'une quelconque des revendications 1 à 11, dans lequel le cadre de retenue comprend en outre un connecteur (322) définissant un passage à travers le cadre de retenue configuré pour recevoir une canule.

13. Système de pompe à perfusion pouvant être portée par l'utilisateur, comprenant :
une pompe à perfusion pouvant être portée par l'utilisateur configurée pour contenir un médicament ; et
le support de pompe de l'une quelconque des revendications 1 à 12.
